# EUROPÄISCHE PATENTSCHRIFT

(11) **EP 2 336 066 B1**
(45) Veröffentlichungstag und Bekanntmachung des Hinweises auf die Patenterteilung: **12.03.2014**
(21) Anmeldenummer: 10194919.6
(22) Anmeldetag: 14.12.2010
(51) Int. Cl.: B65H 75/10, B65H 75/12, A61F 13/00, A61F 15/00, A61K 9/70

(54) **Spule mit darauf aufgewickelter beschichteter Folienbahn**
Coil with a rolled up coated sheet of film
Bobine avec une bande de feuille revêtue enroulée

(30) Priorität: 16.12.2009 DE 102009054803
(43) Veröffentlichungstag der Anmeldung: 22.06.2011
(73) Patentinhaber: Acino AG, 83714 Miesbach (DE)
(72) Erfinder: Ay, Mustafa, 83059, Kolbermoor (DE)
(74) Vertreter: Beetz & Partner

(56) Entgegenhaltungen:
- EP-A2- 1 176 110
- WO-A1-01/22999
- DE-A1- 10 250 955
- DE-U1- 8 811 196
- US-A- 2 676 765
- US-A- 4 934 622
- US-A- 5 762 289
- US-A- 5 857 643
- US-A1- 2005 191 337

## Beschreibung

Die vorliegende Erfindung betrifft eine Aufwickelspule zur Lagerung eines Halbfabrikats für die Herstellung eines transdermalen therapeutischen Systems.

Die vorliegende Erfindung bezieht sich auf das technische Gebiet der Herstellung von transdermalen therapeutischen Systemen, bei denen der oder die Wirkstoffe in einer Polymermatrix enthalten sind, die zumeist aus einem selbsthaftenden druckempfindlichen Polymer gebildet ist. Bei der Herstellung solcher in der Regel als Matrixsysteme bezeichneten transdermalen therapeutischen Systeme wird üblicherweise eine homogene Wirkstoff-Klebstoff-Lösung, z. B. mit Hilfe eines Messergießers kontinuierlich auf eine Trägerfolie aufgebracht. Die mit dieser wirkstoffhaltigen Matrix beschichtete Folie wird getrocknet und anschließend mit einer Schutzfolie kaschiert. Das fertige Laminat wird schließlich auf eine Spule aufgewickelt und bis zur Weiterverarbeitung aufbewahrt oder auf der Spule dem nächsten Prozessschritt zugeführt. Solche Systeme sind aus der WO 01/22999 A1 bekannt.

Die derzeit verwendeten Aufwickelspulen besitzen einen zylinderförmigen Spulenkörper, auf dessen radial außenliegender Oberfläche die erste Wicklung der Folienbahn, gebildet von dem Laminat aus Trägerfolie, wirkstoffhaltiger Matrix und Schutzfolie, unter Druck flach aufliegt. Bei einer Matrix mit hohem Flächengewicht, beispielsweise einer dicken und/oder weichen Matrix, führt dieser Druck zu einer Verdrängung des Matrixmaterials mit dem Resultat einer ungleichmäßigen Verteilung der Matrix auf der Trägerfolie und einem Austreten von Matrixmaterial aus dem Laminat. In der Folge variiert das Flächengewicht eines aus dem aufgewickelten Laminat hergestellten transdermalen therapeutischen Systems in unzulässiger Weise, so dass ein hoher Ausschuss in Kauf genommen werden muss. Außerdem stellt das Austreten der wirkstoffhaltigen Matrix aus dem Laminat sowohl für alle Personen, die potentiell damit in Kontakt kommen können, als auch für die zur Weiterverarbeitung verwendeten Anlagen, ein hohes Kontaminationsrisiko dar.

Ausgehend von dem Dargelegten ist es daher wünschenswert, eine Spule anzugeben, die ein Aufwickeln einer mit einer Matrix beschichteten Trägerfolie ermöglicht, ohne die Verteilung der Matrix auf der Trägerfolie zu beeinträchtigen, insbesondere auch dann, wenn es sich um eine dicke und/oder weiche Matrix handelt.

Diese Aufgabenstellung wird durch die in den beiliegenden Ansprüchen definierten Gegenstände gelöst.

Eine Spule zum Aufwickeln einer beschichteten Folienbahn weist einen zylinderförmigen Spulenkörper und wenigstens zwei ringförmige Stege auf, wobei die Stege an der Oberfläche des Spulenkörpers in einem Abstand zueinander so angeordnet sind, dass die Folienbahn beim Aufwickeln von den Stegen gestützt wird.

Hierdurch wird der beim Aufwickeln auf das Laminat ausgeübte Druck auf die über den Stegen angeordneten Teilbereiche beschränkt, so dass auf die Matrix zwischen den Stegen kein Druck ausgeübt wird, der zu einer Veränderung ihrer Verteilung auf der Trägerfolie führen könnte. Die Stege sind vornehmlich in den Bereichen angeordnet, an denen die Trägerfolie aus produktionstechnischen Gründen nicht mit dem Matrixmaterial belegt ist, und nur der nicht über den Stegen angeordnete Teil des Laminats wird zur Herstellung der transdermalen therapeutischen Systeme verwendet, womit die Schwankungen im Flächengewicht dieser Pflaster minimal und im zulässigen Bereich gehalten werden können.

Bei Ausführungsformen sind die Stege auf dem Spulenkörper so angeordnet, dass die Folienbahn beim Aufwickeln in ihren beiden Randbereichen gestützt wird, womit die größtmögliche durchgängige Fläche zur Herstellung der transdermalen therapeutischen Systeme aus der Folienbahn verfügbar ist. Vorzugsweise sind die über den Stegen angeordneten Bereiche der Trägerfolie nicht mit der wirkstoffhaltigen Matrix beschichtet, so dass die Matrix auch in ihren Randbereichen nicht vom Aufwickeln beeinträchtigt wird.

In vielen Fällen wird die Trägerfolie mit mehreren in Längsrichtung der Trägerfolie parallel zueinander verlaufenden Matrixbeschichtungen versehen. Zum schonenden Aufwickeln einer solchen Folienbahn, aber auch zum schonenden und gleichzeitigen Aufwickeln mehrerer Folienbahnen nebeneinander weist eine Spule gemäß einer weiteren Ausführungsform wenigstens einen weiteren ringförmigen Steg auf, der an der Oberfläche des Spulenkörpers zum Stützen der Folienbahn bzw. der Folienbahnen angeordnet ist. Die Breite jedes der Stege ist an die jeweilige Stützaufgabe angepasst und kann von der eines jeden anderen Stegs abweichen. Vorzugsweise sind die Stege so am Spulenkörper angeordnet, dass sie die Trägerfolie bzw. das Laminat in den nicht mit einer Matrix versehenen Bereichen stützen.

An der Oberfläche des zylinderförmigen Spulenkörpers ist in dem jeweiligen Raum zwischen den Stegen eine hülsenförmige Auflage vorgesehen, die so ausgeführt ist, dass der von der Auflage auf die Folienbahn ausgeübte Druck geringer ist als der von den Stegen auf die Folienbahn ausgeübte Druck. Die hülsenförmige Auflage verhindert eine Formveränderung des Laminats im Bereich zwischen den Stegen, beispielsweise ein über die Aufwickelspannung induziertes Wölben des Laminats oder Faltenbildungen in der Trägerfolie, ohne auf das Laminat einen für die Homogenität der Matrixverteilung auf der Trägerfolie schädlichen Druck auszuüben.

Gemäß einer Ausführungsform kann die hülsenförmige Auflage einen Außendurchmesser aufweisen, der geringfügig kleiner ist als der Außendurchmesser der Stege. Hierdurch konzentriert sich der Aufwickeldruck auf den Stützbereich über den Stegen, wobei spannungsbedingte Formveränderungen des Laminats von der Auflage gleichzeitig innerhalb verträglicher Grenzen gehalten werden. Die Unterschiede in den Außendurchmessern von Stegen und Auflage bzw. Auflagen liegen vorteilhaft in der Größenordnung der Dicke des Laminats.

Die hülsenförmige Auflage kann nach einer weiteren Ausführungsform vollständig oder zumindest an ihrer radial außen liegenden Oberfläche aus einem Material bestehen, das eine höherer Elastizität aufweist als die Stege. Dadurch kann die Oberfläche der Auflage einem auf sie ausgeübten Druck nachgeben und dadurch die Druckbelastung der auf ihr liegenden Folienbahn vermindern.

Bei einer weiteren Ausführungsform weist die Auflage einen geringfügig kleineren Durchmesser als die Stege und eine gegenüber diesen höhere Elastizität an der äußeren Mantelfläche auf, wodurch einer Wellenbildung der Folienbahn beim Aufwickeln wirksam begegnet werden kann. Durchmesser und Elastizität der hülsenförmigen Auflage können vom Fachmann durch einfache Versuche in Abhängigkeit der Eigenschaften der aufzuwickelnden Folienbahn, beispielsweise der Breite der beschichteten Folienbahn, der verwendeten Matrix und deren Eigenschaften wie z.B. Dicke und/oder Fließfähigkeit, der als Schutzfolie bzw. Trägerfolie verwendeten Materialien und deren Eigenschaften wie Dicke und Steifigkeit, so bestimmt werden, dass der Druck auf der zwischen den ringförmigen Stegen liegenden Beschichtungsfläche ausreichend gering ist, damit die Matrix nicht beeinträchtigt wird, und dennoch ausreicht, das Laminat soweit zu stützen, dass keine Falten- oder Wellenbildung auftritt.

Ausführungsformen der Spule umfassen Auflagen bzw. Auflagen mit Oberflächen aus elastisch verformbaren Kunststoffen, und insbesondere aus Schaumkunststoffen mit zelliger Struktur und geeigneten Elastizitätseigenschaften. Insbesondere können die Auflagen bzw. deren Oberflächen unter Verwendung von Polyurethanschäumen (PUR-Schäumen), Polystyrolschäumen (PS-Schäumen) und Schaumstoffen auf der Basis von Natur- oder Synthesekautschuk gebildet sei. Es können beispielsweise Polyurethanschäume verwendet werden, die unter der Handelsbezeichnung Cellasto^{®} von der Firma BASF als Plattenware im Handel erhältlich sind.

Die Stege sind vorzugsweise aus einem Material gebildet, das eine hohe Festigkeit und Formbeständigkeit aufweist, gut verarbeitet werden kann und an dessen Oberfläche ein eventuell aus der Matrix ausgetretener Wirkstoff nicht oder zumindest nicht in einem wesentlichen Umfang adsorbieren kann, beispielsweise aus Polyoxymethylen (POM) oder Polyetheretherketonen (PEEK).

Nach einer bevorzugten Ausführungsform sind die Stege lösbar mit dem zylinderförmigen Spulenkörper verbunden, so dass ihre Anordnung auf dem Spulenkörper in einfacher Weise an die jeweils aufzuwickelnde Folienbahn bzw. Folienbahnen angepasst werden kann. Insbesondere kann hierdurch die Lage der Stege entsprechend der Anordnung der Matrixbeschichtung bzw. -beschichtungen auf der bzw. den Trägerfolien eingestellt werden. Nach einer Ausführungsform können die Stege daher beispielsweise durch Schrauben oder andere geeignete lösbare Verbindungstechniken an dem Spulenkörper befestigt sein.

Vorteilhaft ist der Spulenkörper ferner an einer Seite mit einem Führungselement ausgestattet, das in radialer Richtung einen größeren Radius als der Spulenkörper aufweist und beim Aufwickeln der Folienbahn als Anschlag dienen kann.

Die Erfindung umfasst ein Verfahren zur Herstellung eines transdermalen therapeutischen Systems, das Schritte aufweist zum Herstellen einer Folienbahn durch Aufbringen einer Matrix, die einen oder mehrere Wirkstoffe enthalten kann, auf eine Trägerfolie, Aufwickeln der Folienbahn auf eine wie oben beschriebene Spule, Abrollen der Folienbahn und Vereinzeln des transdermalen therapeutischen Systems aus der Folienbahn. Vor dem Aufwickeln der beschichteten Folienbahn auf die Spule kann diese mit einer Schutzfolie versehen werden.

Die Erfindung bezieht sich auch auf die Verwendung der Spule für die Lagerung eines bahnförmigen Folienlaminats aus einer Trägerfolie, einer gegebenenfalls wirkstoffhaltigen Matrix und einer Schutzfolie bei der Herstellung einer transdermalen therapeutischen Systems bzw. auf ein entsprechendes Verfahren zum Aufbringen eines solchen bahnförmigen Laminats durch Aufwickeln auf eine wie oben beschriebene Spule.

Weitere Merkmale der Erfindung ergeben sich aus der folgenden Beschreibung von Ausführungsbeispielen in Verbindung mit den Ansprüchen sowie den Figuren. Die einzelnen Merkmale können bei einer Ausführungsform gemäß der Erfindung je für sich oder zu mehreren verwirklicht sein. Bei der nachfolgenden Erläuterung einiger Ausführungsbeispiele der Erfindung wird auf die beiliegenden Figuren Bezug genommen, von denen
- Figur 1: eine schematische Darstellung einer ersten Ausführungsform einer Spule zum Aufwickeln einer beschichteten Folienbahn und
- Figur 2: eine schematische Darstellung einer weiteren Ausführungsform einer Spule zum Aufwickeln einer beschichteten Folienbahn in einer Projektionsdarstellung zeigt.

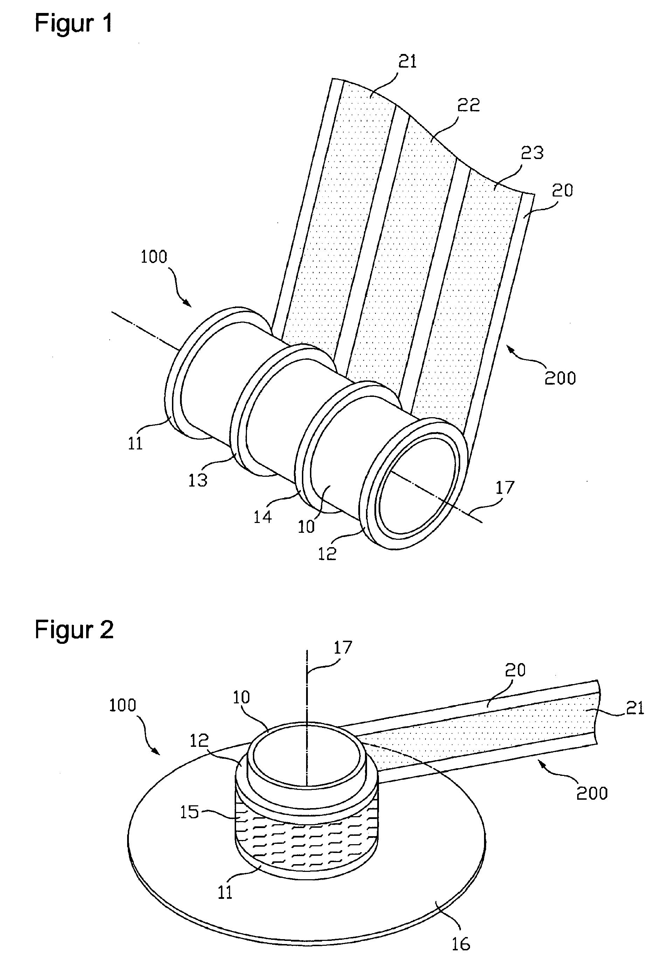

Die schematische Darstellung von Figur 1 veranschaulicht eine Aufwickelspule 100 sowie einen Teil einer zum Aufwickeln auf die Spule vorgesehenen beschichteten Folienbahn 200 in einer Projektionsdarstellung. Figur 1 zeigt nur die Komponenten, die für das Verständnis der vorliegenden Erfindung wesentlich sind. Von einer Darstellung weiterer Komponenten, die für die Funktion der Spule erforderlich sind oder deren Funktionsumfang erweitern, wurde im Hinblick auf eine übersichtliche Darstellung verzichtet. Dennoch sind diese Komponenten als vorhanden angenommen.

Die Spule 100 umfasst einen Spulenkörper 10, der vorzugsweise zylinderförmig ausgeführt ist. An der äußeren Mantelfläche des Spulenkörpers 10 sind wenigstens zwei ringförmige Stege mit vorzugsweise zylinderförmigen Umfangsflächen angeordnet. Die radialen Abmessungen der äußeren Umfangsflächen der Stege sind dabei größer als die der Mantelfläche des Spulenkörpers 10 und weisen an jeder Stelle einen Abstand zu dieser auf.

In der in Figur 1 dargestellten Ausführungsform ist der zylinderförmige Spulenkörper 10 mit vier ringförmigen Stegen 11, 12, 13 und 14 versehen. Die Stege 11 und 12 sind an den bezüglich der Wickelachse 17 der Spule 100 äußeren Rändern des Spulenkörpers 10 angeordnet, dessen axiale Länge im vorgestellten Beispiel der Breite der zum Aufwickeln auf die Spule 100 vorgesehenen Folienbahn 200 entspricht. Zwei weitere Stege 13 und 14 sind auf der Mantelfläche des Spulenkörpers 10 zwischen den beiden äußeren Stegen 11 und 12 angeordnet. Diese Stege dienen dem Stützen der Folienbahn 200 zwischen deren Randzonen.

Abstand und Breite der einzelnen Stege bestimmen sich nach den Eigenschaften der auf die Spule 100 aufzuwickelnden Folienbahn 200. Bei dem in Figur 1 veranschaulichten Beispiel weist das Folienbahnlaminat 200 (die Schutzfolie des Laminats ist nicht dargestellt) eine Trägerfolie 20 auf, auf der die wirkstoffhaltige Matrix in Form von drei parallel zueinander in Längsrichtung der Trägerfolie verlaufenden Bahnen 21, 22 und 23 aufgebracht ist. Die (in der Figur schattiert dargestellten) Matrixbahnen 21, 22 und 23 sind voneinander und von den Rändern der Trägerfolie 20 durch nicht mit einer Matrix beschichtete, streifenförmige Bereiche beabstandet.

Die Breiten der Umfangsflächen der Stege 11, 12, 13 und 14 sind vorzugsweise schmäler als die jeweilige Breite dieser streifenförmigen Bereiche, so dass die Stege die Folienbahn nur in den nicht mit einer Matrix beschichteten Bereichen stützen. Anders ausgedrückt werden die Matrixbahnen 21, 22 und 23 beim Aufwickeln nur um die zwischen den Stegen 11, 12, 13 und 14 ausgebildeten Bereiche geführt. Selbstverständlich können die beiden äußeren Stege breiter ausgeführt sein, um einen über die Folienbahnränder hinausreichenden Überstand zu bilden.

In dem in Figur 1 vorgestellten Beispiel sind die Stege 11, 12, 13 und 14 gleichmäßig beabstandet. Bei Folienbahnen 200 mit unterschiedlich breiten Matrixbahnen wird jedoch eine an die jeweiligen Breiten der Matrixbahnen angepasste ungleichförmige Beabstandung der Stege bevorzugt. In Figur 1 ist eine Folienbahn mit drei Matrixbahnen 21, 22 und 23 dargestellt. Selbstverständlich bestehen bezüglich der Anzahl der Matrixbahnen auf einem aufzuwickelnden Laminat und damit für die Anzahl der auf dem Spulenkörper 10 angeordneten Stege keine Beschränkungen. Weist die Folienbahn 200 jedoch nur eine Matrixbahn auf, so wird die Verwendung von zwei Stegen bevorzugt, die die Folienbahn beim Aufwickeln auf die Spule 100 in ihren Randbereichen stützen.

Bei einer Ausführungsform ist die Spule 100 zum Aufwickeln einer Folienbahn 200 ausgebildet, die eine Breite von 200 mm und drei gleichbreite Matrixbahnen 21, 22 und 23 aufweist. Die axiale Länge der Spule 100 entspricht mit 200mm der Breite der Folienbahn 200. Die Breite der Matrixbahnen auf der Trägerfolie der Folienbahn beträgt typischerweise zwischen 42 und 44 mm. Zum Stützen der Folienbahn 200 in den nicht mit der wirkstoffhaltigen Matrix beschichteten Bereichen weisen die Stege 11, 12, 13 und 14 jeweils eine Breite von 11 mm auf. Der Abstand zwischen den Stegen beträgt 52 mm, so dass die Stege die unbeschichteten Streifen der Folienbahn 200 mittig und mit einem Abstand zu den Rändern der Matrixbahnen 21, 22 und 23 stützen. Hierdurch wird sichergestellt, dass die Stege keinen unmittelbaren Druck auf eine mit der Matrix beschichtete Fläche ausüben.

Die Spule 100 kann einstückig ausgebildet sein, beispielsweise durch Gießen in einer Form oder mithilfe eines spanenden Verarbeitungsverfahrens wie z.B. Drehen aus einem rohr- oder stabförmigen Material. Werden Spulenkörper 10 und Stege 11, 12, und eventuell 13, 14 und unter Umständen weitere Stege separat gefertigt, so werden die Stege in den durch den jeweiligen Anwendungsfall vorgegebenen Abständen auf der äußeren Mantelfläche des Spulenkörpers befestigt, beispielsweise durch Kleben oder Schrauben oder andere geeignete Verbindungstechniken.

In Figur 2 ist eine Ausführungsform dargestellt, bei der zwischen den Stegen 11 und 12 (bei Verwendung mehrerer Stege selbstverständlich auch zwischen diesen Stegen) eine Auflage 15 angeordnet ist. Geometrie und/oder Material der Auflage sind so ausgebildet, dass der von der Auflage 15 auf die Folienbahn 200 ausgeübte Druck geringer als der von den Stegen auf die Folienbahn 200 ausgeübte Druck ist. Die Auflage 15 ist vorzugsweise hülsenförmig ausgebildet, d.h. als den Spulenkörper 10 umhüllendes rohrförmiges Gebilde. Der äußere Querschnitt der Auflage(n) 15 quer zur Wickelachse entspricht dem der Stege, kann jedoch mit etwas kleineren Dimensionen ausgeführt sein. Bei einer zylindrischen oder fassförmigen Ausführung der Umfangsflächen der Stege ist die äußere Umfangsfläche der Auflage vorzugsweise zylindrisch mit einem Durchmesser ausgeführt, der dem maximalen Durchmesser der Stege entspricht oder geringfügig kleiner ist. In bestimmten Fällen kann die Auflage leicht gewölbt sein, beispielsweise um einer ungleichmäßigen Druckverteilung aufgrund einer durch Zugspannungen bedingten Wölbung der Folienbahn 200 vorzubeugen.

Entspricht die äußere Querschnittsform der Auflage 15 senkrecht zur Wickelachse 17 der der Stege, so besteht die Auflage oder zumindest der Oberflächenbereich der Auflage aus einem elastischen Material, das sich unter Druck elastisch verformt und somit den Auflagedruck der Folienbahn reduziert. Da die Folienbahn an der Auflage 15 anliegt, wird einer Faltenbildung wirksam vorgebeugt. Das elastische Material ist vorzugsweise unter den Elastomeren und insbesondere Schaumkunststoffen, wie beispielsweise Polyurethanschäumen (PUR-Schäumen) und Polystyrol (PS-Schäumen), Schaumstoffen auf der Basis von Natur- oder Synthesekautschuk und dergleichen ausgewählt. Der Elastizitätsmodul des elastischen Materials liegt vorzugsweise unter etwa 1 GPa, besonders bevorzugt unter 10⁻¹ GPa und insbesondere im Bereich von 5 · 10⁻³ bis 10⁻² GPa. Um den Auflagedruck der Folienbahn zusätzlich zu verringern, kann die Auflage 15 mit einem etwas kleineren Außendurchmesser als die Stege ausgeführt sein. Vorzugsweise liegen die Unterschiede der Außendurchmesser in der Größenordnung der Folienbahndicke. Das Ausgeführte gilt sinngemäß natürlich auch für nicht-rotationssymmetrische Spulenformen. Bei einer anderen Ausführungsform ist die Auflage nichtelastisch ausgeführt, wobei die Umfangsabmessungen der Auflage bei dieser Ausführungsform stets kleiner gehalten sind als die der Stege.

Bei einer weiteren Ausführungsform des Spulenkörpers, der in Figur 2 dargestellt ist, weist die Spule 100 ein Führungselement 16 auf, das an einer äußeren Stirnseite eines der Stege, hier an der äußeren Stirnfläche des Stegs 11, bzw. am Spulenkörper 10 angeordnet ist. Das scheibenförmige Führungselement 16 führt einen Rand der Folienbahn 200 so, dass jede Wicklungslage der Folienbahn genau über der darunterliegenden angeordnet ist, und die Stege somit nicht auf einen Matrixbereich drücken können. Eine weitere Ausführungsform weist zwei Führungselemente 16, deren Abstand der Breite der Folienbahn eventuell mit einem kleinen Spiel entspricht.

Die Erfindung ermöglicht ein Aufwickeln einer mit einer wirkstoffhaltigen Matrix beschichteten Trägerfolie ohne Beeinträchtigung der Matrix. Insbesondere gewährleistet die Erfindung ein Aufrechterhalten eines gleichmäßigen Flächengewichts der Matrixbeschichtung und verhindert ein Austreten der Matrix aus dem Folienverbund. In der Folge verringert sich der Ausschuss bei der Herstellung von transdermalen therapeutischen Systemen aus der Folienbahn und die Gefahr einer Kontamination von Herstellungsanlagen oder Personen. Die Spule eignet sich daher insbesondere zur Aufnahme eines Folienverbundes aus einer Trägerfolie, einer gegebenenfalls wirkstoffhaltigen Matrix und einer Schutzfolie, um diesen Folienverbund bei der Herstellung eines transdermalen therapeutischen Systems bis zur Weiterverarbeitung aufzubewahren oder dem nächsten Prozessschritt zuzuführen.

Zur Herstellung von transdermalen therapeutischen Systemen mit gleichmäßig verteiltem, d.h. homogenen, und konstanten Flächengewicht der Matrixbeschichtung wird zunächst die Polymermatrix 21, 22, 23, die einen oder mehrere Wirkstoffe enthalten kann, auf eine als Trägerfolie dienende Folienbahn 20 aufgebracht. Zum Auftragen der Matrix kann z. B. ein Messergießer eingesetzt werden. Wie in Fig. 1 dargestellt ist, kann die Beschichtung in Matrixbahnen 21, 22, 23 erfolgen, die durch nicht beschichtete Bereiche voneinander getrennt sind.

Die beschichtete Folienbahn 200 kann zusätzlich mit einer Schutzfolie kaschiert werden. Diese Schutzfolie kann als ablösbare Schutzschicht für das transdermale therapeutische System dienen oder im Laufe des weiteren Verfahrens durch eine solche ablösbare Schutzschicht ersetzt werden.

Die beschichtete Folienbahn 200 wird auf eine wie oben beschriebene Spule 100 aufgewickelt. In einem nachfolgenden Arbeitsschritt wird die beschichtete Folienbahn von der Spule 100 abgewickelt und mit bekannten Verfahren wie z.B. Stanzen, in einzelne transdermale therapeutische Systeme vereinzelt.

Hierbei können die ausgestanzten Teile der beschichteten Folienbahn auf die ablösbare Schutzschicht übertragen werden, falls nicht die Schutzfolie selbst als ablösbare Schutzschicht dient.

Selbstverständlich werden die transdermalen therapeutischen Systeme nur aus den Bereichen der beschichteten Folienbahn 200 gefertigt, die während der Aufbewahrung auf der Spule 100 nicht über den Stegen 11, 12, 13, 14 angeordnet waren.

## Patentansprüche

1. Verfahren zur Herstellung eines transdermalen therapeutischen Systems mit den folgenden Schritten:
- Herstellen einer Folienbahn (200) durch Aufbringen einer wirkstoffhaltigen Matrix auf eine Trägerfolie;
- Aufwickeln der Folienbahn (200) auf eine Spule (100);
- Abrollen der Folienbahn (200) und Vereinzeln des transdermalen therapeutischen Systems aus der Folienbahn (200),
wobei die Spule (100) einen zylinderförmigen Spulenkörper (10) und wenigstens zwei ringförmige Stege (11, 12) aufweist, die an der Oberfläche des Spulenkörpers (10) und in einem Abstand zueinander so angeordnet sind, dass die Folienbahn (200) beim Aufwickeln von den Stegen (11, 12) gestützt wird, und **dadurch gekennzeichnet ist, dass** in dem Raum zwischen den wenigstens zwei (11, 12) Stegen eine hülsenförmige Auflage (15) angeordnet und so ausgebildet ist, dass der von der Auflage (15) auf die Folienbahn (200) ausgeübte Druck geringer ist als der von den Stegen (11, 12, 13, 14) auf die Folienbahn (200) ausgeübte Druck.

2. Verfahren nach Anspruch 1, worin die Stege (11, 12) der Spule (100) auf dem Spulenkörper (10) in einem solchen Abstand zueinander angeordnet sind, dass die Folienbahn (200) beim Aufwickeln in ihren beiden Randbereichen gestützt wird.

3. Verfahren nach Anspruch 1 oder 2, worin die Spule (100) einen oder mehrere weitere ringförmige Stege (13, 14) aufweist, die an der Oberfläche des Spulenkörpers (10) zum Stützen der Folienbahn (200) angeordnet sind.

4. Verfahren nach einem der Ansprüche 1 bis 3, worin bei der Spule (100) der Außendurchmesser der hülsenförmigen Auflage (15) kleiner ist als der Außendurchmesser der Stege (11, 12, 13, 14) und/oder die radial außen liegende Oberfläche der hülsenförmige Auflage (15) aus einem Material gebildet ist, das eine höhere Elastizität aufweist als das zur Ausbildung der Stege (11, 12, 13, 14) verwendete Material.

5. Verfahren nach Anspruch 4,worin das Material der hülsenförrnigen Auflage (15) der Spule (100) unter Schaumkunststoffen und insbesondere Schaumstoffen auf der Basis von Natur- / Synthetikkautschuken, Polyurethanschäumen und Polystyrolschäumen ausgewählt ist.

6. Verfahren nach einem der vorangehenden Ansprüche, worin das Material der Stege (11, 12, 13, 14) ausgewählt ist unter Polyoxymethylen (POM) oder Polyetheretherketonen (PEEK).

7. Verfahren nach einem der vorhergehenden Ansprüche, worin die Spule (100) ferner ein Führungselement (16) aufweist, das an einer Seite des Spulenkörpers (10) angebracht ist und in radialer Richtung einen größeren Radius aufweist als der Spulenkörper (10).

8. Verfahren nach einem der vorangehenden Ansprüche, worin die Folienbahn (200) vor dem Aufwickeln auf die Spule (100) mit einer Abdeckfolie kaschiert wird.

9. Spule mit darauf aufgewickelter beschichteter Folienbahn für die Herstellung eines transdermalen therapeutischen Systems erhältlich durch Aufwickeln einer beschichteten Folienbahn auf eine Spule (100), wobei die beschichtete Folienbahn durch Aufbringen einer wirkstoffhaltigen Matrix auf eine Trägerfolie hergestellt ist und die Spule (100) Folgendes aufweist:
i) einen zylinderförmigen Spulenkörper (10) und
ii) wenigstens zwei ringförmige Stege (11, 12), die an der Oberfläche des Spulenkörpers (10) und in einem Abstand zueinander so angeordnet sind, dass die Folienbahn (200) beim Aufwickeln von den Stegen (11, 12) gestützt wird,
**dadurch gekennzeichnet, dass**
in dem Raum zwischen den wenigstens zwei (11, 12) Stegen eine hülsenförmige Auflage (15) angeordnet und so ausgebildet ist, dass der von der Auflage (15) auf die Folienbahn (200) ausgeübte Druck geringer ist als der von den Stegen (11, 12, 13, 14) auf die Folienbahn (200) ausgeübte Druck.

10. Spule mit darauf aufgewickelter beschichteter Folienbahn nach Anspruch 9, wobei die Spule (100) ferner Merkmale nach einem der Ansprüche 2 bis 7 aufweist.

11. Verwendung einer Spule mit darauf aufgewickelter beschichteter Folienbahn nach Anspruch 9 für die Lagerung einer beschichten Folienbahn, die aus einer Trägerfolie, einer gegebenenfalls wirkstoffhaltigen Matrix und einer Schutzfolie gebildet ist.

12. Verwendung nach Anspruch 11, worin die Spule (100) ferner Merkmale nach einem der Ansprüche 2 bis 7 aufweist.

## Claims

1. Method for producing a transdermal therapeutic system comprising the following steps:
- producing a film web (200) by applying an active substance-containing matrix to a backing film;
- winding the film web (200) onto a spool (100);
- unwinding the film web (200) and separating the transdermal therapeutic systems from the film web,
the spool (100) having a cylindrical spool body (10) and at least two annular ridges (11, 12) which are arranged on the surface of the spool body (10) and at a mutual spacing such that film web (200) is supported by the ridges (11, 12) during winding, and **characterised in that** a sleeve-shaped support (15) is arranged in the space between the at least two (11, 12) ridges and is formed such that the pressure exerted on the film web (200) by the support (15) is lower than the pressure exerted on the film web (200) by the ridges (11, 12, 13, 14).

2. Method according to claim 1, wherein the ridges (11, 12) of the spool (100) are arranged on the spool body (10) at such a mutual spacing that the film web (200) is supported in its two edge regions during winding.

3. Method according to either claim 1 or claim 2, wherein the spool (100) has one or more additional annular ridges (13, 14) which are arranged on the surface of the spool body (10) to support the film web (200).

4. Method according to any of claims 1 to 3, wherein, in the spool (100), the outer diameter of the sleeve-shaped support (15) is smaller than the outer diameter of the ridges (11, 12, 13, 14) and/or the radially outer surface of the sleeve-shaped support (15) is formed from a material which has a higher elasticity than the material used to form the ridges (11, 12, 13, 14).

5. Method according to claim 4, wherein the material of the sleeve-shaped support (15) of the spool (100) is chosen from among foamed plastics and in particular foams on a base of natural or synthetic rubber, polyurethane foams and polystyrene foams.

6. Method according to any of the preceding claims, wherein the material of the ridges (11, 12, 13, 14) is chosen from among polyoxymethylene (POM) or polyether ether ketones (PEEK).

7. Method according to any of the preceding claims, wherein the spool (100) also has a guide element (16) which is attached to one side of the spool body (10) and has a larger radius in the radial direction than the spool body (10).

8. Method according to any of the preceding claims, wherein the film web (200) is laminated with a sealing film before being wound onto the spool (100).

9. Spool, comprising a coated film web wound thereon, for producing a transdermal therapeutic system which is obtained by winding a coated film web onto a spool (100), the coated film web being produced by applying an active substance-containing matrix onto a backing film, said spool (100) having the following:
i) a cylindrical spool body (10) and
ii) at least two annular ridges (11, 12) which are arranged on the surface of the spool body (10) and at a mutual spacing such that the film web (200) is supported by the ridges (11, 12) during winding, **characterised in that**
a sleeve-shaped support (15) is arranged in the space between the at least two (11, 12) ridges and is formed such that the pressure exerted on the film web (200) by the support (15) is lower than the pressure exerted on the film web (200) by the ridges (11, 12, 13, 14).

10. Spool comprising a coated film web wound thereon according to claim 9, wherein the spool (100) also has features according to any of claims 2 to 7.

11. Use of a spool comprising a coated film web wound thereon according to claim 9 for storing a coated film web which is formed from a backing film, a matrix optionally containing an active substance, and a protective film.

12. Use according to claim 11, in which the spool (100) also has features according to any of claims 2 to 7.

## Revendications

1. Procédé de fabrication d'un système thérapeutique transdermique, comprenant les étapes suivantes :
- fabrication d'une bande de feuille (200) par l'application, sur une feuille support, d'une matrice contenant un principe actif ;
- enroulement de la bande de feuille (200) sur une bobine (100) ;
- déroulement de la bande de feuille (200) et découpe du système thérapeutique transdermique à partir de la bande de feuille (200),
la bobine (100) présentant un corps de bobine (10) de forme cylindrique et au moins deux nervures (11, 12) annulaires qui sont disposées sur la surface du corps de bobine (10) et de façon espacée entre elles de telle façon que, lors de l'enroulement, la bande de feuille (200) est supportée par les nervures (11, 12), et **caractérisé en ce que**, dans l'espace entre les au moins deux nervures (11, 12), un appui (15) en forme de douille est disposé et constitué de telle façon que la pression exercée par l'appui (15) sur la bande de feuille (200) est plus faible que la pression exercée par les nervures (11, 12, 13, 14) sur la bande de feuille (200).

2. Procédé selon la revendication 1, dans lequel les nervures (11, 12) de la bobine (100) sont disposées sur le corps de bobine (10) avec une telle distance entre elles que, lors de l'enroulement, la bande de feuille (200) est supportée au niveau de ses deux zones de bord.

3. Procédé selon la revendication 1 ou 2, dans lequel la bobine (100) présente une ou plusieurs autres nervures (13, 14) annulaires qui sont disposées sur la surface du corps de bobine (10) pour supporter la bande de feuille (200).

4. Procédé selon une des revendications 1 à 3, dans lequel, au niveau de la bobine (100), le diamètre extérieur de l'appui (15) en forme de douille est plus petit que le diamètre extérieur des nervures (11, 12, 13, 14) et/ou la surface, située à l'extérieur radialement, de l'appui (15) en forme de douille est formée d'un matériau qui présente une élasticité plus élevée que le matériau utilisé pour la constitution des nervures (11, 12, 13, 14).

5. Procédé selon la revendication 4, dans lequel le matériau de l'appui (15) en forme de douille de la bobine (100) est sélectionné parmi des mousses de matière plastique et en particulier des mousses à base de caoutchoucs naturels / synthétiques, des mousses de polyuréthane et des mousses de polystyrène.

6. Procédé selon une des revendications précédentes, dans lequel le matériau des nervures (11, 12, 13, 14) est sélectionné parmi le polyoxyméthylène (POM) ou des polyétheréthercétones (PEEK).

7. Procédé selon une des revendications précédentes, dans lequel la bobine (100) présente en outre un élément de guidage (16) qui est mis en place sur un côté du corps de bobine (10) et qui présente, dans la direction radiale, un rayon plus grand que le corps de bobine (10).

8. Procédé selon une des revendications précédentes, dans lequel, avant l'enroulement sur la bobine (100), la bande de feuille (200) est doublée avec une feuille de couverture.

9. Bobine sur laquelle est enroulée une bande de feuille revêtue, pour la fabrication d'un système thérapeutique transdermique pouvant être obtenu par l'enroulement d'une bande de feuille revêtue sur une bobine (100), la bande de feuille revêtue étant fabriquée par l'application d'une matrice contenant le principe actif sur une feuille support, et la bobine (100) présentant les éléments suivants :
i) un corps de bobine (10) de forme cylindrique et
ii) au moins deux nervures (11, 12) annulaires qui sont disposées sur la surface du corps de bobine (10) et de façon espacée entre elles de telle façon que, lors de l'enroulement, la bande de feuille (200) est supportée par les nervures (11, 12),
**caractérisée en ce que**,
dans l'espace entre les au moins deux nervures (11, 12), un appui (15) en forme de douille est disposé et constitué de telle façon que la pression exercée par l'appui (15) sur la bande de feuille (200) est plus faible que la pression exercée par les nervures (11, 12, 13, 14) sur la bande de feuille (200).

10. Bobine sur laquelle est enroulée une bande de feuille revêtue selon la revendication 9, la bobine (100) présentant en outre des caractéristiques selon une des revendications 2 à 7.

11. Utilisation d'une bobine sur laquelle est enroulée une bande de feuille revêtue selon la revendication 9, pour supporter une bande de feuille revêtue qui est formée d'une feuille support, d'une matrice contenant éventuellement le principe actif et d'une feuille de protection.

12. Utilisation selon la revendication 11, dans laquelle la bobine (100) présente en outre des caractéristiques selon une des revendications 2 à 7.
